# EUROPEAN PATENT SPECIFICATION

(11) **EP 2 512 493 B1**
(45) Date of publication and mention of the grant of the patent: **19.08.2015**
(21) Application number: 10795525.4
(22) Date of filing: 10.12.2010
(51) Int. Cl.: A61K 35/22, A61P 13/12

(54) **METHOD OF RENAL REPAIR AND REGENERATION AND THE TREATMENT OF DIABETIC NEPHROPATHY**
VERFAHRNE ZUR NIERENREPARATUR UND -REGNERATION SOWIE BEHANDLUNG VON DIABETISCHER NEPHROPATHIE
PROCÉDÉ DE RÉPARATION ET DE RÉGÉNÉRATION DU TISSU RÉNAL ET TRAITEMENT DE LA NÉPHROPATHIE DIABÉTIQUE

(30) Priority: 15.12.2009 US 286421 P
(43) Date of publication of application: 24.10.2012
(73) Proprietor: DePuy Synthes Products, Inc., Raynham, MA 02767 (US)
(72) Inventor: COLTER, David, C., Hamilton NJ 08610 (US); KAZANECKI, Christian, C., Martins Creek PA 18063 (US)
(74) Representative: Wise, Daniel Joseph
(86) International application number: PCT/US2010/059816
(87) International publication number: WO 2011/081841

(56) References cited:
- WO-A1-2008/045498
- BRUNO S ET AL: "Isolation and Characterization of Resident Mesenchymal Stem Cells in Human Glomeruli", STEM CELLS AND DEVELOPMENT, MARY ANN LIEBERT, INC, NEW ROCHELLE, NY, US, vol. 18, no. 6, 29 June 2009 (2009-06-29), pages 867-880, XP002567261, ISSN: 1557-8534, DOI: DOI:10.1089/SCD.2008.0320 [retrieved on 2009-02-02]

## Description

### FIELD OF THE INVENTION

The disclosure relates to cell-based therapies for the treatment of chronic kidney disease. In particular, the disclosure relates to the use of human kidney derived cells (hKDCs) for the treatment of diabetic nephropathy.

### BACKGROUND OF THE INVENTION

Diabetic nephropathy is a leading cause of end-stage renal disease, and its incidence is increasing worldwide. Thirty to forty percent of insulin-dependent diabetes mellitus patients suffer from end-stage diabetic nephropathy, which develops 35 to 40 years after the onset of diabetes. Once diabetic nephropathy becomes overt, there is no curative therapy, and most patients eventually progress to end-stage renal disease.

Kidney disease is a serious, unmet medical condition with an annual U.S. cost burden exceeding $27 billion. Currently, more than 40 million Americans are at risk for or have kidney disease, and the incidence is increasing at an alarming rate of 6% per year. Therefore, by the year 2020, an estimated one in four people will have end-stage renal disease (ESRD), requiring either dialysis or kidney transplantation. To alleviate these economic and medical challenges, transformational technologies for the treatment of chronic kidney disease (CKD) are necessary.

CKD is a gradual and progressive loss of kidney function. It is generally irreversible and ultimately leads to end-stage renal disease. In the United States, CKD is becoming increasingly common and is associated with poor health outcomes and high medical costs. The National Kidney Foundation estimates that 20 million Americans have CKD, and at least 20 million additional people are at risk for developing CKD. If left untreated, CKD can lead to significant morbidity and mortality from anemia, electrolyte imbalances, bone disease, cardiovascular disease, and kidney failure.

Progressive chronic renal disease results from a combination of the initial disease injury (*e.g*., hypertension), followed by a maladaptive renal response to that injury. Such a response includes the production of pro-inflammatory and pro-fibrotic cytokines and growth factors. Therefore, one strategy to slow CKD progression is to ameliorate the inflammatory and fibrotic response as well as repair or reverse existing kidney damage. It has been shown that the administration of growth factors can slow CKD progression. For example, bone morphogenic protein-7 (BMP-7) prevented tubular atrophy, interstitial inflammation and fibrosis in rats with unilateral ureteric obstruction. Similarly, BMP-7 administration reduced tubulointerstitial fibrosis and glomerulosclerosis in a mouse model of lupus nephritis. In addition, hepatocyte growth factor has been shown to have potent anti-inflammatory and anti-fibrotic efficacy in a wide variety of animal models of kidney injury. Other factors that have shown therapeutic promise include transforming growth factor-β1, vascular endothelial growth factor (VEGF), connective tissue growth factor, fibroblast growth factor-2 (FGF-2), interleukins, tumor necrosis factor, and monocyte chemotactic protein-1. Multipotent mesenchymal stromal cells (MSCs) have been shown to contribute to tissue regeneration in injured bone and cartilage, as well as in the infarcted heart, brain, and kidney. Ezquer et al.(Biology of Blood and Marrow Transplantation 14:631-640, 2008) have suggested that MSCs might also contribute to pancreas and kidney regeneration in diabetic individuals. Ezquer et al. (Biol Blood Marrow Transplant 15:1354-1365, 2009) also showed that MSCs can prevent renal failure in diabetic mice. In addition, Semedo et al. (Mesenchymal Stem Cells Attenuate Renal Fibrosis Through Immune Modulation and Remodeling Properties in a Rat Remnant Kidney Model. Stem Cells Empress, Sept 2009*)* tested the hypothesis that multipotent stromal cells from human bone marrow can slow the progression of renal fibrosis. These studies all demonstrate that the administration of cells is a promising therapeutic approach for the preventative treatment of CKD.

Despite existing medical treatment options, mortality rates remain very high and the incidence of kidney disease is on the rise. Therefore, a need exists for an improved, potentially curative therapy. Today, no therapeutic intervention attempts to halt or even reverse the progression of diabetic nephropathy.

### SUMMARY OF THE INVENTION

The invention relates to a population of human kidney derived cells for use in a method for treating diabetic nephropathy comprising administering a therapeutic amount of 0.18 x 10⁶ to 1.00 x 10⁶ of the human kidney derived cells to a mammalian subject; wherein the human kidney-derived cells are capable of self-renewal and expansion in culture; wherein the human kidney derived cells are positive for the cell surface marker expression of HLA I and CD44, and for the gene expression of Oct-4, Pax-2, and WT1; and wherein the human kidney derived cells are negative for the cell surface marker expression of CD 133, and the gene expression of Wnt-4.

### BRIEF DESCRIPTION OF THE FIGURES

**Figure 1****:** Implant preparation: hKDC were seeded onto GELFOAM (Pharmacia) punches and cultured for seven days in rotating bioreactor vessels. Empty scaffold (A). hKDC seeded scaffold (B). Images were obtained using a dissecting microscope at 7X magnification (A) or 12.5x magnification (B).
**Figure 2****:** Protein excretion rate: Total protein excretion rate was determined for normal mice as well as for GELFOAM treated animals and hKDC-loaded GELFOAM treated animals on day 0 and day 14 post-transplantation. Error bars represent SEM. * = p = 0.05.
**Figure 3****:** Blood glucose: Legend shows animal treatment group number described in Table 8. Error bars represent standard deviation.
**Figure 4****:** Urine glucose excretion rate: Urine glucose excretion rate was determined for normal mice compared to treated animals on day 0 and day 28 post-transplantation. Error bars represent SEM. Treatment groups are described in Table 7.
**Figure 5****:** Total protein excretion rate: Total protein excretion rate was determined for normal mice compared to treated animals on day 0 and day 28 post-transplantation. Error bars represent SEM. Treatment groups are described in Table 7. * = p < 0.002.
**Figure 6****:** Survival Rate: Survival rate was determined in a normal SCID mouse model and compared to diabetic SCID mouse model with and without cell treatments as described in Table 11.

### DETAILED DESCRIPTION OF THE INVENTION

The invention is defined in the claims.

Various terms relating to the methods and other aspects of the invention are used throughout the specification and claims. Such terms are to be given their ordinary meaning in the art unless otherwise indicated.

The disclosure relates to a method of treating diabetic nephropathy in a mammalian subject which comprises administering to the mammalian subject a therapeutically effective amount of isolated human kidney derived cells.

The isolated human kidney derived cells are isolated as described in US Patent Publication Number 2008/0112939. The human kidney derived cells are isolated from a human kidney that is suitable for organ transplantation. Briefly, blood and debris are removed from the kidney by washing with any suitable medium or buffer such as phosphate buffered saline. Human kidney derived cells are then isolated from the kidney by mincing the kidney and then followed by enzymatic digestion. Combinations of collagenase, dispase, and hyaluronidase are used to dissociate cells from the human kidney tissue. Isolated cells are then transferred to sterile tissue culture vessels that are initially coated with gelatin. Human kidney derived cells are cultured in any culture medium capable of sustaining growth of the cells such as, but not limited to, renal epithelial growth medium (REGM).

Human kidney derived cells are passaged to a separate culture vessel containing fresh medium of the same or a different type as that used initially, where the population of cells can be mitotically expanded. The cells may be used at any point between passage 0 and senescence. In one embodiment, the cells are passaged between about 3 to about 20 times. In another embodiment, the cells are passaged between about 4 to about 20 times. In yet another embodiment, the cells are passaged between about 10 to about 11times. Human kidney derived cells are characterized by phenotypic characteristics, for example, morphology, growth potential, surface marker phenotype, early development gene expression, kidney development gene expression and trophic factor secretion. Surface marker, gene expression and trophic factor secretion phenotype is retained after multiple passages of the human kidney derived cells in culture.

An isolated human kidney derived cell is capable of self-renewal and expansion in culture, wherein the cell is positive for expression of at least one of Oct-4, Rex-1, Pax-2, Cadherin-11, FoxD1, WT1, Eyal, HNF3B, CXC-R4, Sox-17, EpoR, BMP2, BMP7, or GDF5 and negative for the expression of at least one of Sox2, FGF4, hTert, Wnt-4, SIX2 or GATA-4. The cell is positive for expression of at least one of Eyal, WT1, FoxD1, BMP7, BMP2, GDF5, EpoR or Rex-1, and negative for expression of at least one of Sox2, FGF4, hTert or Wnt-4. The human kidney derived cell is also positive for at least one of cell-surface markers HLA I, CD24, CD29, CD44, CD49c, CD73, CD166, or SSEA-4, and negative for at least one of cell-surface markers HLA II, CD31, CD34, CD45, CD56, CD80, CD86, CD104, CD105, CD117, CD133, CD138, CD141, or E-cadherin. The cell is preferably non-immunogenic for allogeneic transplantation in a mammalian subject. The human kidney derived cell may secrete at least one of trophic factors FGF2, HGF, TGFα, TIMP-1, TIMP-2, MMP-2 or VEGF. Preferably, the cell does not secrete at least one of trophic factors PDGF-bb or IL12p70.

We define a therapeutically effective amount of human kidney derived cells to mean a dosage of cells that halts or reverses the progression of chronic kidney disease. The halting or reversal of the progression of diabetic nephropathy may be demonstrated by a reduction in total protein excretion rate in mammals with diabetic nephropathy. It addition, a therapeutically effective amount of cells will slow the progress of renal injury and will delay the on-set of end-stage renal failure. In one embodiment, a therapeutically effective amount of cells is about 0.18e6 to about 1.00e6. In one example, 0.18e6 hKDCs, seeded onto a GELFOAM scaffold, slowed the progression of diabetic nephropathy in a transgenic model of diabetes. In another example, 1.00e6 hKDCs, delivered under the kidney capsule, caused a reduction in the rate of total protein excretion compared to vehicle treatment in an STZ model of diabetes.

The cells are locally administered by any suitable method for administering cell therapies. Suitable methods for local administration of human kidney derived cells include, but are not limited to transplanting, implanting, injecting, infusing, and the like. In one embodiment, the cells are administered to the kidney. In another embodiment the cells are administered to the kidney capsule of a kidney. In yet another embodiment the cells are administered to the subcapsular region of the kidney.

In one embodiment, the human kidney derived cells are administered to the mammalian subject by local injection or infusion. The cells are combined with a suitable carrier for ease of injection or infusion. Suitable carriers for injection include, but are not limited to water, buffered saline, phosphate buffer solution, Hank's balanced salts solution, Tris buffered saline, Hepes buffered saline and the like. Optionally, the cells could be formulated with viscosity enhancers such as alginate, hyaluronic acid, collagen gel, fibrin glue, fibrin clot, poly(N-isopropylacrylamide), agarose, chitin, chitosan, cellulose, polysaccharides, poly(oxyalkylene), a copolymer of poly(ethylene oxide)-poly(propylene oxide), poly(vinyl alcohol), polyacrylate, platelet rich plasma (PRP) clot, platelet poor plasma (PPP) clot, Matrigel, blood clot, gelatin-resorcin-formalin adhesives, mussel-based adhesives, dihydroxyphenylalanine (DOPA) based adhesives, transglutaminase, poly(amino acid)-based adhesives, cellulose-based adhesives, polysaccharide-based adhesives, synthetic acrylate-based adhesives, liquid and semi-solid fatty acid esters of glycerol and succinic acid (MGSA), MGSA/polyethylene glycol (MGSA/PEG) copolymers, polyvinylpyrolidone (PVP), PVP copolymers, gelatin, albumin, monoglycerides, diglycerides, triglycerides laminin, elastin, proteoglycans, and combinations thereof.

For example, the cells are combined with a saline carrier such as phosphate buffered saline and then the cells are injected directly under the kidney capsule of one or both kidneys. The effective cell dosage in rodents is from about 0.18x10⁶ to about 1.00x10⁶ cells per kidney per rodent. One of skill in the art would be able to extrapolate the effective dosage of cells suitable to treat a human with chronic kidney disease.

In another embodiment, the cells are administered by implantation. The cells may be implanted with or without a suitable scaffold. Suitable scaffolds include, but are not limited to textiles, such as weaves, knits, braids, meshes, and non-wovens; perforated films; sponges and foams; and beads, such as solid or porous beads, microparticles, nanoparticles, and the like. In one embodiment, the scaffold is a foam.

The polymers used to prepare scaffolds described herein are natural polymers, synthetic polymers, and combinations thereof. The polymers are biodegradable and biocompatible. The biodegradable polymers readily break down into small segments when exposed to moist body tissue. The segments then either are absorbed by the body, or passed by the body. More particularly, the biodegraded segments do not elicit permanent chronic foreign body reaction, because they are absorbed by the body or passed from the body, such that no permanent trace or residual of the segment is retained by the body.

Examples of suitable synthetic biocompatible, biodegradable polymers that could be used include polymers selected from the group consisting of aliphatic polyesters, poly(amino acids), copoly(ether-esters), polyalkylene oxalates, polyamides, poly(iminocarbonates), polyorthoesters, polyoxaesters, polyamidoesters, polyoxaesters containing amine groups, poly(anhydrides), polyphosphazenes, biomolecules and blends thereof. For the purpose of this invention aliphatic polyesters include, but are not limited to homopolymers and copolymers of lactide (which includes lactic acid, d-, 1- and meso lactide), glycolide (including glycolic acid), epsilon-caprolactone, p-dioxanone (1,4-dioxan-2-one), trimethylene carbonate (1,3-dioxan-2-one), alkyl derivatives of trimethylene carbonate, delta-valerolactone, beta-butyrolactone, gamma-butyrolactone, epsilon-decalactone, hydroxybutyrate (repeating units), hydroxyvalerate (repeating units), 1,4-dioxepan-2-one (including its dimer 1,5,8,12-tetraoxacyclotetradecane-7,14-dione), 1,5-dioxepan-2-one, 6,6-dimethyl-1,4-dioxan-2-one 2,5-diketomorpholine, pivalolactone, alpha, alpha-diethylpropiolactone, ethylene carbonate, ethylene oxalate, 3-methyl-1,4-dioxane-2,5-dione, 3,3-diethyl-1,4-dioxan-2,5-dione, 6,8-dioxabicycloctane-7-one and polymer blends thereof.

In one embodiment, the scaffold may be prepared from aliphatic polyesters which include, but are not limited to homopolymers and copolymers of lactide (which includes lactic acid, D-,L- and meso lactide), glycolide (including glycolic acid), epsilon-caprolactone, p-dioxanone (1,4-dioxan-2-one), trimethylene carbonate (1,3-dioxan-2-one), alkyl derivatives of trimethylene carbonate, delta-valerolactone, beta-butyrolactone, gamma-butyrolactone, epsilon-decalactone, hydroxybutyrate (repeating units), hydroxyvalerate (repeating units), 1,4-dioxepan-2-one (including its dimer 1,5,8,12-tetraoxacyclotetradecane-7,14-dione), 1,5-dioxepan-2-one, 6,6-dimethyl-1,4-dioxan-2-one and polymer blends thereof.

In another embodiment, the scaffolds may be prepared from natural polymers including, but not limited to collagen, gelatin, chitin, hyaluronic acid, elastin, fibronectin and the like are also suitable for the purposes of this invention. In yet another embodiment, the scaffold is prepared from gelatin.

The cells can be grown freely in culture, removed from the culture and seeded onto a scaffold. For example, a one centimeter long by four millimeter wide collagen based foam scaffold is seeded with human kidney derived cells in a rotating bioreactor or equivalent system. The cell seeded scaffold is then cultured for seven days and then surgically implanted under the kidney capsule of the mammalian subject.

Optionally, the human kidney derived cells can be administered in sequence with or co-administered with one or more biologically active agents, such as antithrombogenic agents, anti-apoptotic agents, anti-inflammatory agents, immunosuppressants (*e.g.*, cyclosporine, rapamycin), antioxidants, or other agents ordinarily used in the art to treat kidney damage. Such agents include, but are not limited to eprodisate and triptolide.

Optionally, the human kidney derived cells can be administered in sequence with, or co-administered with the other cells or agents. For example, biological agents generated from cells, such as lysates, soluble cell fractions, membrane-enriched cell fractions, cell culture media (*e.g*., conditioned media), or extracellular matrix from human kidney derived cells can also be administered to patients as appropriate, including co-administered with human kidney derived cells themselves, and additional cells or agents. Therapeutic cells can be co-administered with cell lysates, or with other allogeneic, syngeneic or autologous cells. In some aspects, it is useful to re-create in culture the cellular microenvironment found *in vivo,* such that the extent to which the cells are grown prior to implantation *in vivo* or used *in vitro* may vary. The cells can be seeded onto the scaffold before or after forming the shape desired for implantation, *e.g.*, ropes, tubes, filaments, meshes and the like. Following seeding of the cells onto the scaffold, the scaffold is preferably incubated in an appropriate growth medium. During the incubation period, the cells will grow and envelop the scaffold and may for example bridge, or partially bridge any interstitial spaces therein. It is preferable, but not required to grow the cells to an appropriate degree which reflects the *in vivo* cell density of the kidney tissue being repaired or regenerated. In other embodiments, the presence of the cells, even in low numbers on the scaffold encourages in-growth of endogenous healthy cells to facilitate healing for example of the damaged or injured tissue.

Optionally , the external surfaces of the scaffold can be modified to improve the attachment or growth of cells and differentiation of tissue, such as by plasma coating the scaffold or addition of one or more proteins (*e.g*., collagens, elastic fibers, reticular fibers), glycoproteins, glycosaminoglycans (*e.g*., heparin sulfate, chondroitin-4-sulfate, chondroitin-6-sulfate, dermatan sulfate, keratin sulfate), a cellular matrix, and/or other materials such as, but not limited to, gelatin, alginates, agar, agarose, and plant gums, among others.

The scaffold can be comprised of or treated with materials that render it non-thrombogenic. These treatments and materials may also promote and sustain endothelial growth, migration, and extracellular matrix deposition. Examples of these materials and treatments include but are not limited to natural materials such as basement membrane proteins such as laminin and type IV collagen, synthetic materials such as ePTFE, and segmented polyurethaneurea silicones. These materials can be further treated to render the scaffold non-thrombogenic. Such treatments include anti-thrombotic agents such as heparin, and treatments which alter the surface charge of the material such as plasma coating.

The methods described herein have utility for treating chronic kidney disease, including diabetic nephropathy resulting in morbidity or reduced life expectancy. Treatment with human kidney derived cells slows the progression of diabetic nephropathy and therefore will delay the onset of end-stage renal disease.

The following examples are provided to describe the invention in greater detail. They are intended to illustrate, not to limit, the invention.

### REFERENCE EXAMPLE 1

The renoprotective efficacy of a locally administered hKDC loaded collagen foam scaffold was evaluated in a transgenic model of diabetic nephropathy. The collagen foam scaffold is sold under the tradename GELFOAM (Pharmacia and Upjohn Co., Kalamazoo, MI).

### Animal model

Eighteen week old, male C57BL/6-Ins2^{Akita} (Akita) and C57BL/6 control mice were obtained from The Jackson Laboratories (Bar Harbor, ME). Ins2^{Akita} is a model of type 1 diabetes. The Akita spontaneous mutation is an autosomal dominant mutation in the insulin II gene (Ins2). This missense mutation results in an amino acid substitution (cysteine 96 to tyrosine), which corresponds to the seventh amino acid position of the insulin II A chain. The mice were maintained on a 12-h light/dark cycle and fed standard mouse chow.

### Cell Preparation

A healthy human kidney was obtained from the National Disease Research Interchange (NDRI, Philadelphia, PA). Tissue was dissected from the outer cortex region of the kidney. The tissues were then mechanically dissociated in tissue culture plates. The tissue was then transferred to a 50-milliliter conical tube. The tissue was then digested with an enzyme mixture containing 0.25 units PZ activity/mL collagenase (NB6, N0002779; Serva Electrophoresis GmbH, Heidelberg, Germany), 2.5 units/mL dispase (Dispase II 165 859, Roche Diagnostics Corporation, Indianapolis, IN), 1 unit/mL hyaluronidase (Vitrase, ISTA Pharmaceuticals, Irvine, Ca). The enzyme mixture was combined with renal epithelial growth medium (REGM) (Lonza, Walkersville, MA). The conical tubes containing the tissue, medium and digestion enzymes were incubated at 37°C in an orbital shaker at 225 rpm for 1-2 hours.

The digested material was filtered through a 40-micron nylon BD FALCON Cell strainer (BD Biosciences, San Jose, CA). The filtrate was resuspended in REGM (total volume 50 milliliters) and centrifuged at 150 x g for 5 minutes. The supernatant was aspirated and the cells were resuspended in 50 milliliters of REGM. This process was repeated twice.

After the final centrifugation, supernatant was aspirated and the cell pellet was resuspended in 10 milliliters of REGM. Cells were then plated at a seeding density of 10,000 cells/cm² onto gelatin-coated tissue culture flasks and cultured at 37°C under normal atmospheric conditions. Passage seven cells were cryopreseved and subsequently used in this study.

**Table 1. Experimental design. Untreated C57BL/6-Ins2^{Akita} (Akita) diabetic mice were used as the sham control. No scaffolds were implanted (Group 1), GELFOAM scaffold only, without cells (Group 2), GELFOAM scaffold seeded with hKDC (Group 3). Urine was collected using metabolic cages over twelve-hour duration on day 6 and 27-post transplantation.**

| **Treatment group** | **Description** | **Number of animals** | **Urine collection/ Necropsy (Day)** |
|---|---|---|---|
| 1 | Sham | 2 | 6 and 27/27 |
| 2 | GELFOAM only | 3 | 6 and 27/27 |
| 3 | GELFOAM + hKDC | 5 | 6 and 27/27 |

### Implant Preparation

GELFOAM was cut into small pieces using a 2 mm biopsy and then placed into 100% ethanol for approximately 30 minutes. GELFOAM punches were then washed two times with 1 mL phosphate buffered saline (Gibco). hKDC were thawed at 37°C and resuspended at 4e⁶ hKDCs per 10 mL of REGM media. Each cell suspension was then deposited into a rotating bioreactor vessel (Sythecon, Inc, Houston, TX) with eight GELFOAM punches per bioreactor. Implants were then cultured for six days with media exchange every 1-3 days. Negative control implants (Group 2) were cultured in the same manner as cell-loaded implants. The day before transplantation, cell seeded and negative control implants were then transferred to 1.5 mL cryovials containing 1 mL of respective growth medium. Implants were then shipped to the testing facility. After arrival, implants were placed into a 37°C incubator, until the time of transplantation. To confirm the viability and cell dosage of the implants, four implants per treatment group, and two GELFOAM only, negative control implants were evaluated for morphological integrity by light microscopy. In addition, implants were digested with trypsin and both cell number and viability was determined using a Guava Instrument (Guava technologies, Hayward CA).

### Transplantation

At the time of transplantation, growth media was decanted from the tubes containing the implants and the implants were washed two times in HBSS (Hanks Balanced Salt Solution w/o Ca⁺⁺/Mg⁺⁺) and reconstituted in 1 mL of HBSS. With the mouse under light anesthesia, the left kidney was exposed through a flank incision. A capsulotomy was performed in the lower kidney pole using a 23-gauge needle, and either GELFOAM alone or cell-loaded GELFOAM was gently implanted. The capsulotomy was then cauterized with a disposable low-temperature cautery device. The muscle was closed with sutures and the skin stapled. The mice were allowed to recover under a warm lamp and then returned to their normal housing (Surgicare, Dayville, CT).

### Urine Collection and Analysis

Mice were weighed and blood glucose was determined. All animals had blood glucose of over 600mg/dl (One Touch Ultra II, Lifescan). Each animal was put in a separate metabolic cage (Lab products Inc) for 24 hrs. The cage system holds up to 8 mice. All animals had unlimited access to food and water. After 24hrs, the animals were returned to their normal housing. The urine was lightly centrifuged to remove any debris, collected with a pipette to determine the volume and then frozen. Urine was collected on day 6 and 27 post-transplantation. The Urine was then analyzed for creatinine and protein concentrations. Creatinine excretion was measured using ADVIA 1650 System (Bayer Corporation, Tarrytown, NY). Total protein excretion was measured using a Bayer Clinitek Atlas Urine Chemistry Analyzer (Bayer Corporation, Elkhart, IN).

### Necropsy and Histology

At day 27 after the transplant and after urine collection, the animals were anesthsized with Ketamine and Xylazine. The mice were opened up and the left kidney exposed. Once the scaffold was identified, the kidney was excised and trimmed to have the scaffold oriented up in the histology cassette. Kidneys were then fixed for 4 hours in fresh 4% paraformaldehyde. After fixing kidneys were stored in PBS at 4 degrees C until embedding. The kidneys were embedded in paraffin and sections were made at 4-5um. One section of each kidney was stained with H&E and one for Vimentin (1:100 overnight at 4 degrees C, clone V9, DAKO).

### Results

### Implant Characterization

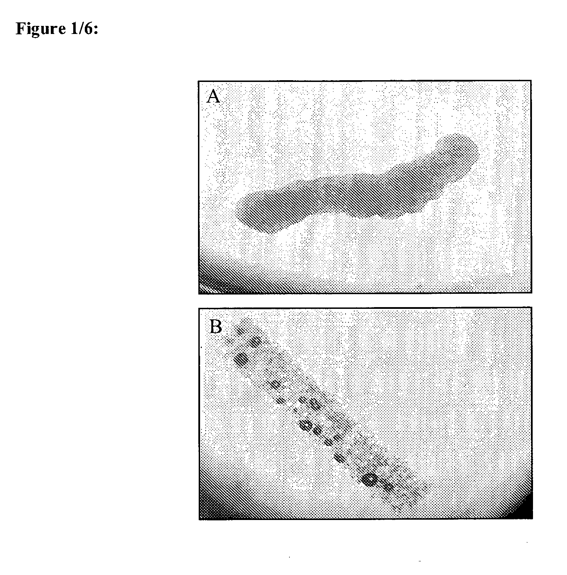

Figure 1 demonstrates that hKDC loaded GELFOAM implants are intact with robust architectural integrity. In addition, the implants have a consistent and uniform length and width. Cell loaded implants showed some shrinkage compared to negative control implants.

To determine cell dosage and viability of the implants prior to implantation, 4 implants were digested with trypsin and harvested cells were evaluated using a Guava instrument (Table 2). Results showed that hKDC-loaded implants have an average number of viable cells of 106,553 +/- 19,261 with an average viability of 89% +/- 2%.

### Urine Chemistry

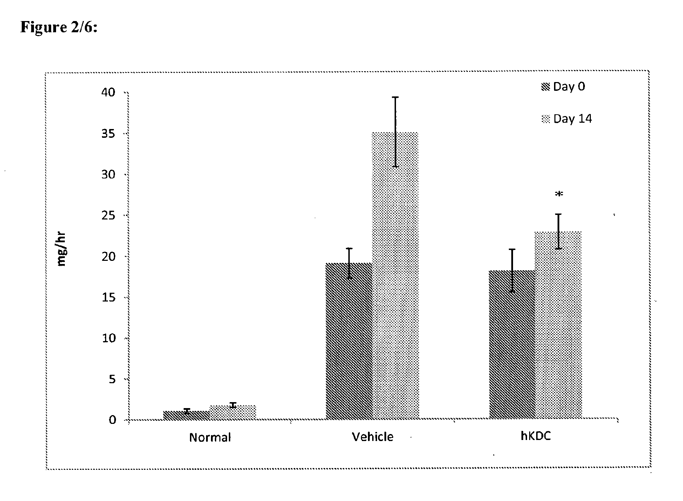

On day 6 and day 27 post transplantation, urine was collected for twelve hours from all animals and analyzed for both creatinine and protein excretion. Table 3 and Figure 2 demonstrates that both creatinine and protein excretion decreased over the twenty-seven day study duration. However, there was no statistical difference in creatinine or protein excretion between GELFOAM alone and cell-loaded GELFOAM treated animals.

### Histology

On day 27-post transplantation, all left kidneys were evaluated for the presence of implant. No GELFOAM implants were identified in any of the kidneys.

### Conclusion

Data demonstrates that local transplantation of human kidney derived cells can be achieved using GELFOAM scaffold. GELFOAM can be seeded with hKDC using a rotating bioreactor culture system and the resulting cell-loaded implant has a robust and uniform morphology. In addition, cell attachment to the scaffold is consistent and cell viability is high. Therefore, cell-seeded GELFOAM is a potentially valuable therapeutic concept for delivering cells to an injured tissue or organ.

Despite the successful construction of the GELFOAM implant, its ability to improve renal function in a rodent model of diabetic nephropathy was not demonstrated in this example. In addition, it appears that the GELFOAM completely degraded after twenty-seven days under the kidney capsule. This might explain the resulting lack of renoprotective efficacy. In future studies, it may be important to evaluate renal function at earlier time points as the effect could be transient in this animal model. In addition, hKDC of an earlier passage might be more effective. It may also be important to evaluate other renal function parameters (i.e. total protein excretion rate) so that renal injury can be fully characterized. Maintenance of GELFOAM integrity might be an important factor for maintaining a high therapeutic cell dose within the injured kidney environment, and therefore observing GELFOAM integrity and human cell persistence at earlier time points is necessary.

**Table 2. Implant dosage and viability analysis. Implants were digested with trypsin and cell number and viability was evaluated using a Guava instrument.**

| Implant number | Viable cells | Viability (%) |
|---|---|---|
| **Mean** | **106,533** | **89** |
| **Std** | **19261** | **2** |

**Table 3: Urine analysis: Urine was collected over twelve-hour duration and then analyzed for creatinine and protein excretion. See Table 1 for treatment group description.**

| **Day** | **Group** | Avg | Std | Avg | Std |
|---|---|---|---|---|---|
| 6 | 1 | 2.03 | 0.63 | 16.41 | 16.04 |
| 6 | 2 | 2.74 | 0.34 | 26.35 | 3.20 |
| 6 | 3 | 2.55 | 0.17 | 24.48 | 3.41 |
| 27 | 1 | 0.85 | 0.25 | 7.35 | 4.71 |
| 27 | 2 | 0.92 | 0.27 | 9.74 | 4.08 |
| 27 | 3 | 0.90 | 0.07 | 10.30 | 2.14 |

### EXAMPLE 2

In this study, the methods of Example 1 were repeated except that we increased the cell dosage on the hKDC loaded GELFOAM scaffold implant. In addition, the urine protein excretion rate over the course of fourteen days, in the Akita transgenic mouse model of diabetic nephropathy was evaluated.

### Methods

### Animal model

Eighteen week old, male C57BL/6-Ins2^{Akita} and C57BL/6 control mice were obtained from The Jackson Laboratories Ins2^{Akita} is a model of type 1 diabetes. The Akita spontaneous mutation is an autosomal dominant mutation in the insulin II gene (Ins2). The mice were maintained on a 12-h light/dark cycle and fed standard mouse chow.

### Cell Preparation

A healthy human kidney was obtained from the National Disease Research Interchange Tissue was dissected from the outer cortex region of the kidney. The tissues were then mechanically dissociated in tissue culture plates until minced to a fine pulp. The tissue was then transferred to a 50-milliliter conical tube. The tissue was then digested an enzyme mixture containing 0.25 units PZ activity/mL collagenase (NB6, N0002779; Serva Electrophoresis GmbH, Heidelberg, Germany), 2.5 units/mL dispase (Dispase II 165 859, Roche Diagnositics Corporation, Indianapolis, IN), 1 unit/mL hyaluronidase (Vitrase, ISTA Pharmaceuticals, Irvine, Ca). The enzyme mixture was combined with REGM. The conical tubes containing the tissue, medium and digestion enzymes were incubated at 37°C in an orbital shaker at 225 rpm for 1-2 hours.

The digested material was filtered through a 40-micron nylon BD FALCON Cell strainer (BD Biosciences). The filtrate was resuspended in REGM (total volume 50 milliliters) and centrifuged at 150 x g for 5 minutes. The supernatant was aspirated and the cells were resuspended in 50 milliliters of REGM. This process was repeated twice.

After the final centrifugation, supernatant was aspirated and the cell pellet was resuspended in 10 milliliters of REGM. Cells were then plated at a seeding density of 10,000 cells/cm² onto gelatin-coated tissue culture flasks and cultured at 37°C under normal atmospheric conditions. Passage three cells were cryopreseved and subsequently used in this study. The experimental design is described in Table 1.

**Table 4. Experimental design. Untreated, normal C57BL/6 mice (Group 1). C57BL/6-Ins2^{Akita} diabetic mice were treated with GELFOAM only, without cells (Group 2). GELFOAM seeded with hKDC (Group 3). Urine was collected using metabolic cages over twelve-hour duration on day 0 and day 14-post transplantation.**

| **Treatment group** | **Animal number** | **Description** | **Number of animals** | **Urine collection/ Necropsy (Day)** |
|---|---|---|---|---|
| 1 | 21-24 | Normal C57/BL6 mice | 4 | 0 and 14/14 |
| 2 | 13-20 | GELFOAM only | 8 | 0 and 14/14 |
| 3 | 1-12 | GELFOAM + hKDC | 12 | 0 and 14/14 |

### Implant Preparation

GELFOAM was cut into small pieces using a 4 mm biopsy and then placed into 100% ethanol for approximately 30 minutes. GELFOAM punches were then washed two times with 1 mL phosphate buffered saline (Gibco). HKDC (passage 3) were thawed at 37°C and resuspended at 4.9e⁶ hKDC per 10 mL of REGM. The cell suspension was then deposited into a rotating bioreactor vessel (Sythecon Inc, Houston, TX) with eight GELFOAM punches per bioreactor. Implants were cultured at 37°C overnight, with rotation at 20 rpm and then media was exchanged. Implants were then cultured for an additional six days. Negative control implants (Group 2) were cultured in the same manner as cell-loaded implants. The day before transplantation, cell seeded and negative control implants were then transferred to 1.5 mL cryovials containing 1 mL of REGM. Implants were then shipped to the study site by same-day courier service. After arrival, implants were placed into a 37°C incubator, until the time of transplantation. To confirm the viability and cell dosage of the implants, four implants were evaluated for morphological integrity by light microscopy. In addition, implants were digested with trypsin and both cell number and viability was determined using a Guava Instrument (Guava technologies) (Table 5).

### Transplantation

At the time of transplantation, growth media was decanted from the tubes containing the implants and the implants were washed two times in HBSS and reconstituted in 1 mL of HBSS. With the mouse under light anesthesia, the left kidney was exposed through a flank incision. A capsulotomy was performed in the lower kidney pole using a 23-gauge needle, and either GELFOAM alone or cell-loaded GELFOAM was gently implanted. The capsulotomy was then cauterized with a disposable low-temperature cautery device. The muscle was closed with sutures and the skin stapled. The mice were allowed to recover under a warm lamp and then returned to their normal housing (Surgicare,).

### Urine Collection and Analysis

Mice were weighed and blood glucose was determined. All animals had blood glucose of over 600 mg/dl (One Touch Ultra II, Lifescan). Each animal was put in a separate metabolic cage for twenty-four hours. The cage system holds up to 8 mice. All animals had unlimited access to food and water. After twelve hours, the animals were returned to their normal housing. The urine was lightly centrifuged to remove any debris, collected with a pipette to determine the volume and then frozen. Urine was collected on day 0 and 14 post-transplantation and analyzed.

### Results

### Implant Characterization

To determine cell dosage and viability of the implants, 4 implants were digested with trypsin and harvested cells were evaluated using a Guava instrument (Table 5). Results showed that hKDC-loaded implants have an average number of viable cells of 176,850 +/- 65,578 with a viability of 96% +/- 2.

### Urine Chemistry

On day 0 and day 14-post transplantation, urine was collected for twelve hours from all animals and analyzed. Table 6 demonstrates that hKDC treatment had little effect on creatinine excretion, total urine creatinine, total urine glucose, urine glucose excretion and total urine protein concentration. However, hKDC treatment did have an effect on the rate of urine protein excretion. Figure 2 demonstrates that on day 0 protein excretion rate was equivalent between hKDC and control treated animals. However, on day fourteen the rate of protein excretion was significantly lower (37%) in the hKDC treatment group compared to control treated animals. Both control animals and hKDC treated animals showed an elevated protein excretion rate compared to normal, non-diabetic animals.

### Conclusion

In the present study, we reduced the study duration from 27 days to 14 days, as well as increased the cell dosage to improve on the methods described in Example 1. We showed that hKDC loaded implants having a cell dosage of approximately 176,000 hKDC per implant can reduce total protein excretion by approximately 37% after 14 days post implantation. In addition, hKDC of an earlier passage were utilized. In Example 1, passage 7 hKDC were tested while we used passage 3 cells here in Example 2. These data do suggest that hKDC have a therapeutic benefit and reduce the degree of renal injury in the diabetic animal. Future studies should focus on the effects of hKDC within another model of diabetic nephropathy.

**Table 5: Implant dosage and viability analysis. Implants were digested with trypsin and cell number and viability was evaluated using a Guava instrument.**

| **Implant number** | **Cell number** | **Viability (%)** |
|---|---|---|
| **Mean** | **176850** | **96** |
| **Stdev** | **65578** | **2** |

**Table 6: Urine chemistry. Urine samples were obtained from all animals and urine chemical analysis was conducted. Refer to Table 4 for treatment group description.**

| | | | | Urine Volume | UCREAT_Excr | UCREAT | UGLU | UGLU_CR | UGLU_Excr | UPROT | UPROT_CR | UPROT_Excr |
|---|---|---|---|---|---|---|---|---|---|---|---|---|
| **Treatment group number** | **Animal number** | **Implant number** | **Day** | **(mL) 24h** | (mg/hr) | (mg/dL) | (mg/dL) | (mg/mg) | (mg/hr) | (mg/dL) | (mg/mg) | (mg/hr) |
| **3** | **1** | 1 | 0 | 23.3 | 1.89 | 8.11 | 8627 | 1063.75 | 2010.09 | 94.5 | 11.65 | 22.02 |
| **3** | **2** | 2 | 0 | 219.3 | 19.23 | 8.77 | 10046 | 1145.50 | 22030.88 | 93.3 | 10.64 | 204.61 |
| **3** | **3** | 3 | 0 | 9.4 | 0.83 | 8.84 | 8074 | 913.35 | 758.96 | 71.9 | 8.13 | 6.76 |
| **3** | **4** | 4 | 0 | 5.2 | 0.56 | 10.68 | 8369 | 783.61 | 435.19 | 97.5 | 9.13 | 5.07 |
| **3** | **5** | 8 | 0 | 12.0 | 0.75 | 6.25 | 7013 | 1122.08 | 841.56 | 41.2 | 6.59 | 4.94 |
| **3** | **6** | 7 | 0 | 19.0 | 1.61 | 8.48 | 9760 | 1150.94 | 1854.40 | 94.2 | 11.11 | 17.90 |
| **3** | **7** | 9 | 0 | 16.3 | 2.05 | 12.58 | 12702 | 1009.70 | 2070.43 | 112.6 | 8.95 | 18.35 |
| **3** | **8** | 10 | 0 | 15.8 | 2.07 | 13.09 | 12361 | 944.31 | 1953.04 | 115.9 | 8.85 | 18.31 |
| **2** | **9** | 17 | 0 | 16.2 | 1.90 | 11.73 | 12549 | 1069.82 | 2032.94 | 102.4 | 8.73 | 16.59 |
| **2** | **10** | 18 | 0 | 22.1 | 2.11 | 9.55 | 9846 | 1030.99 | 2175.97 | 101.6 | 10.64 | 22.45 |
| **2** | **11** | 23 | 0 | 25.0 | 1.91 | 7.62 | 9747 | 1279.13 | 2436.75 | 86.4 | 11.34 | 21.60 |
| **2** | **12** | 24 | 0 | 13.1 | 1.60 | 12.25 | 11460 | 935.51 | 1501.26 | 115.7 | 9.44 | 15.16 |
| **1** | **21** | NA | 0 | 1.2 | 0.19 | 15.82 | 5368 | 339.32 | 64.42 | 109.7 | 6.93 | 1.32 |
| **1** | **22** | NA | 0 | 0.7 | 0.12 | 17.22 | 4976 | 288.97 | 34.83 | 110.5 | 6.42 | 0.77 |
| **1** | **23** | NA | 0 | 0.1 | 0.02 | 23.05 | 4096 | 177.70 | 4.10 | 113.0 | 4.90 | 0.11 |
| **1** | **24** | NA | 0 | 1.1 | 0.65 | 58.73 | 187 | 3.18 | 2.06 | 124.2 | 2.11 | 1.37 |

**Table 6: Urine chemistry (Cont.). Urine samples were obtained from all animals and urine chemical analysis was conducted. Refer to Table 4 for treatment group description.**

| | | | | Urine Volume | UCREAT_Excr | UCREAT | UGLU | UGLU_CR | UGLU_excr | UPROT | UPROT_R | UPROT_Excr |
|---|---|---|---|---|---|---|---|---|---|---|---|---|
| **Treatment group** | **Animal number** | **Implant number** | **Day** | (mL) 24hr | (mg/hr) | (mg/dL) | (mg/dL) | (mg/mg) | (mg/hr) | (mg/dL) | (mg/mg) | (mg/hr) |
| **3** | **1** | 1 | 14 | 42.2 | 3.08 | 7.30 | 8671 | 1187.81 | 3659.16 | 70.1 | 9.60 | 29.58 |
| **3** | **2** | 2 | 14 | 37.0 | 2.55 | 6.88 | 8624 | 1253.49 | 3190.88 | 54.6 | 7.94 | 20.20 |
| **3** | **3** | 3 | 14 | 18.5 | 1.49 | 8.05 | 8408 | 1044.47 | 1555.48 | 78.2 | 9.71 | 14.47 |
| **3** | **4** | 4 | 14 | 36.7 | 2.67 | 7.27 | 8224 | 1131.22 | 3018.21 | 80.4 | 11.06 | 29.51 |
| **3** | **5** | 8 | 14 | 27.5 | 1.93 | 7.00 | 7467 | 1066.71 | 2053.43 | 61.6 | 8.80 | 16.94 |
| **3** | **6** | 7 | 14 | 35.8 | 2.39 | 6.67 | 8811 | 1320.99 | 3154.34 | 59.2 | 8.88 | 21.19 |
| **3** | **7** | 9 | 14 | 26.5 | 2.28 | 8.59 | 10072 | 1172.53 | 2669.08 | 92.5 | 10.77 | 24.51 |
| **3** | **8** | 10 | 14 | 29.8 | 3.05 | 10.22 | 9831 | 961.94 | 2929.64 | 98.9 | 9.68 | 29.47 |
| **2** | **9** | 17 | 14 | 39.6 | 3.85 | 9.71 | 11291 | 1162.82 | 4471.24 | 99.8 | 10.28 | 39.52 |
| **2** | **10** | 18 | 14 | 39.0 | 3.27 | 8.39 | 8900 | 1060.79 | 3471.00 | 96.0 | 11.44 | 37.44 |
| **2** | **11** | 23 | 14 | 37.2 | 2.88 | 7.74 | 8735 | 1128.55 | 3249.42 | 88.0 | 11.37 | 32.74 |
| **2** | **12** | 24 | 14 | 19.3 | 2.04 | 10.58 | 9803 | 926.56 | 1891.98 | 106.9 | 10.10 | 20.63 |
| **1** | **21** | NA | 14 | 1.4 | 0.84 | 59.81 | 56 | 0.94 | 0.78 | 127.2 | 2.13 | 1.78 |
| **1** | **22** | NA | 14 | 0.8 | 0.54 | 66.92 | 72 | 1.08 | 0.58 | 127.8 | 1.91 | 1.02 |
| **1** | **23** | NA | 14 | 1.8 | 1.18 | 65.42 | 84 | 1.28 | 1.51 | 125.9 | 1.92 | 2.27 |
| **1** | **24** | NA | 14 | 1.5 | 1.04 | 69.24 | 146 | 2.11 | 2.19 | 120.9 | 1.75 | 1.81 |

### EXAMPLE 3

The purpose of this study was to evaluate the renoprotective effects of hKDC (without a scaffold) delivered locally under the kidney capsule of a streptozotocine (STZ) SCID mouse model of diabetes.

### Study Design

**Table 7: Experimental design.**

| Treatment group | Number of animals | Treatment |
|---|---|---|
| 1 | 8 | Non-diabetic, no treatment |
| 2 | 8 | STZ + vehicle |
| 3 | 8 | STZ + 1e⁶ hKDC |

### Animal Model

ICR SCID mice at 6-8 weeks of age will be given a single dose (180mg/kg i.p.) of Streptozotocin (Sigma) to induce diabetes. Blood glucoses over 350mg/dl will be considered diabetic. Two weeks after the injection of STZ, cells will be administered into the kidney cortex of diabetic animals.

### Dose Preparation

At the time of transplantation, hKDC were injected under the kidney capsule of mice. The mice were anesthetized with ketamine and Xylazine. The left kidney was exposed and a small incision (0.3 cm) was made in the lower pole of the kidney capsule using a 23g needle. hKDC (1.0e⁶ cells) were injected through a PE 50 (VWR) siliconized tubing attached to a 200ul tip on a Hamilton threaded syringe (Hamilton). The incision was cauterized, the muscle sutured and the skin stapled. The mice were allowed to recover under a warm lamp and then returned to their normal housing.

### Terminal Procedures

### Serum analysis

On day 0 and 28-post cell administration, body weight and blood samples will be obtained from all mice and blood glucose concentration will be measured. All blood glucoses will be checked in the morning for a random fed glucose value. If the animals do achieve normal blood glucoses, at day 28 will an Intraperitoneal Glucose tolerance test (IPGTT) will be conducted. Twelve hr fasted animals will be given a 2g/kg i.p. injection of glucose and blood sugars will be taken at times 0, 15, 30, 60 and 90 minutes.

### Urine analysis

On day 0 and 28 post-transplantation, each animal will be weighed and placed into metabolic cages (Lab products Inc) for 24 hrs. After collection, the animals will be returned to their normal housing. The urine will be lightly centrifuged to remove any debris, collected with a pipette to determine the volume and then frozen. Urine protein, creatinine and glucose excretion will be measured.

### Results

### Serum analysis

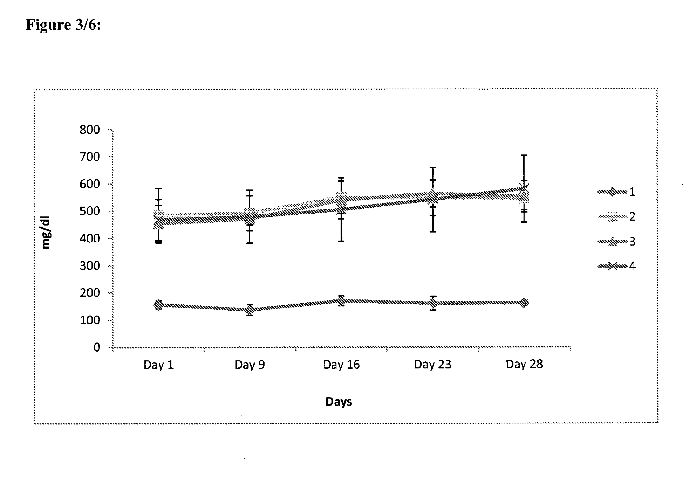

On day 1,9, 16, 23 and 28-post transplantation blood samples were obtained from all mice and blood glucose concentration were measured. Table 8 and Figure 3 shows that blood glucose values were elevated in all animals except non-diabetic animals. Values remained at a constant value throughout the duration of the study. On day 28-post cell transplantation, non-diabetic animals (Group 1) showed mean blood glucose of 161-mg/dL +/- 9 mg/dL. Vehicle treated animals (Group 2) had a mean blood glucose of 545 mg/dL +/- 42 mg/dL. hKDC treated animals (Group 3) had a mean blood glucose of 552 mg/dL +/- 57 mg/dL and hUTC treated animals (Group 4) had a mean blood glucose of 581 mg/dL +/- 123 mg/dL.

### Urine analysis

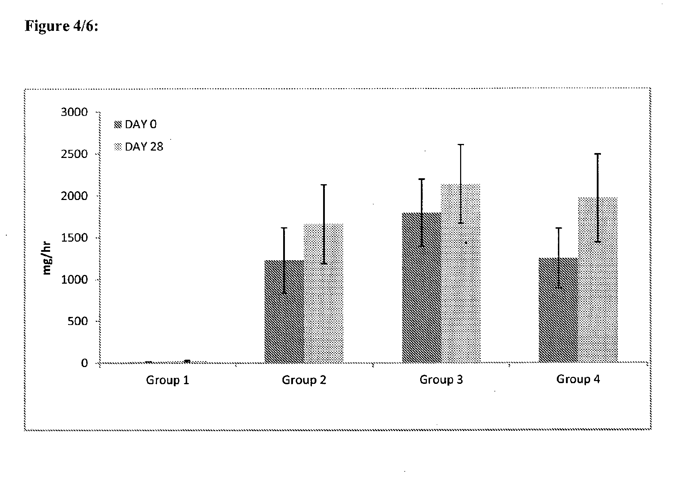

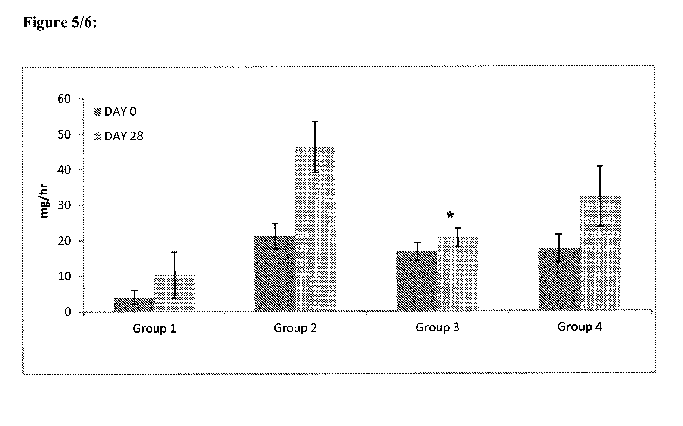

On day 0 and day 28-post transplantation, urine was collected for twenty-four hours from all animals and analyzed. The rate of glucose excretion increases overtime and was similar among all treatment groups (Table 9 and Figure 4). However, compared to non-diabetic animals (Group 1), vehicle treated and cell treated animals showed an elevated rate of protein excretion. In all animals, except hKDC treated animals, the rate of protein excretion increased over time. Compared to vehicle treatment (Group 2), hKDC treatment resulted in a 55% reduction in the rate of total protein excretion (Table 9, Figure 5). On day 28, the rate of total protein excretion was 10.29 mg/hr +/- 6.44 mg/hr for Group 1 animals, 46.29 mg/hr +/- 7.16 mg/hr for Group 2 animals, 20.63 mg/hr +/- 2.65 mg/hr for Group 3 animals and 32.13 mg/hr +/- 8.44 mg/hr for Group 4 animals.

### Conclusion

Previously, we demonstrated that hKDC, seeded into a GELFOAM scaffold is renoprotective in a transgenic mouse model of diabetic nephropathy. Here we confirm our results, as well as demonstrate that local administration of hKDC, without a scaffold, protects the kidney from diabetic injury in an STZ model of diabetes. Data showed that local subcapsular administration of 1.0x06 hKDCs reduces the rate of total protein excretion by 55% in an STZ *SCID* model of diabetes mellitus.

**Table 8: Blood Glucose.**

| | | Day 1 | Day 9 | Day 16 | Day 23 | Day 28 |
|---|---|---|---|---|---|---|
| Group | | (mg/dL) | (mg/dL) | (mg/dL) | (mg/dL) | (mg/dL) |
| 1 | | 142 | 120 | 161 | 149 | 166 |
| 1 | | 158 | 113 | 161 | 124 | 164 |
| 1 | | 141 | 139 | 157 | 146 | 157 |
| 1 | | 162 | 140 | 175 | 175 | 150 |
| 1 | | 181 | 170 | 166 | 187 | 155 |
| 1 | | 154 | 141 | 204 | 182 | 176 |
| | **Mean** | **156** | **137** | **171** | **161** | **161** |
| | **STDEV** | **15** | **20** | **17** | **25** | **9** |
| 2 | | 510 | 583 | 600 | 571 | 578 |
| 2 | | 364 | 439 | 480 | 512 | 511 |
| 2 | | 372 | 455 | 491 | 452 | 507 |
| 2 | | 578 | 278 | 571 | 549 | 600 |
| 2 | | 600 | 538 | 583 | 600 | 529 |
| 2 | | 478 | 449 | 600 | 600 | 600 |
| | **Mean** | **484** | **493** | **551** | **547** | **545** |
| | **STDEV** | **100** | **64** | **60** | **64** | **42** |
| 3 | | 360 | 480 | 422 | 546 | 508 |
| 3 | | 440 | 447 | 429 | 482 | 429 |
| 3 | | 547 | 447 | 600 | 600 | 544 |
| 3 | | 493 | 472 | 527 | 563 | 600 |
| 3 | | 503 | 505 | 600 | 600 | 554 |
| 3 | | 458 | 450 | 476 | 474 | 487 |
| 3 | | 361 | 478 | 541 | 550 | 529 |
| 3 | | 472 | 491 | 496 | 600 | 600 |
| | **Mean** | **454** | **471** | **540** | **565** | **552** |
| | **STDEV** | **66** | **22** | **69** | **51** | **57** |
| 4 | | 600 | 520 | 533 | 581 | 582 |
| 4 | | 363 | 284 | 243 | 244 | 242 |
| 4 | | 501 | 495 | 489 | 470 | 600 |
| 4 | | 514 | 574 | 556 | 575 | 562 |
| 4 | | 411 | 516 | 468 | 517 | 600 |
| 4 | | 489 | 543 | 546 | 600 | 600 |
| 4 | | 455 | 525 | 600 | 600 | 600 |
| 4 | | 409 | 379 | 375 | 490 | 526 |
| | **Mean** | **468** | **480** | **506** | **542** | **581** |
| | **STDEV** | **75** | **98** | **116** | **118** | **123** |

**Table 9: Urine glucose excretion rate.**

| **DAY 0** | **Mean (mg/hr)** | **SEM** |
|---|---|---|
| Group 1 | 12.42 | 4.14 |
| Group 2 | 1226.73 | 390.83 |
| Group 3 | 1793.71 | 399.44 |
| Group 4 | 1251.06 | 355.44 |
| | | |

| **DAY 28** | **Mean (mg/hr)** | **SEM** |
|---|---|---|
| Group 1 | 21.03 | 10.59 |
| Group 2 | 1659.29 | 471.21 |
| Group 3 | 2137.91 | 469.64 |
| Group 4 | 1964.92 | 524.55 |

**Table 10: Total urine protein excretion rate.**

| **DAY 0** | **Mean (mg/hr)** | **SEM** |
|---|---|---|
| Group 1 | 4.00 | 1.99 |
| Group 2 | 21.20 | 3.52 |
| Group 3 | 16.71 | 2.52 |
| Group 4 | 17.56 | 3.84 |
| | | |

| **DAY 28** | **Mean (mg/hr)** | **SEM** |
|---|---|---|
| Group 1 | 10.29 | 6.44 |
| Group 2 | 46.29 | 7.16 |
| Group 3 | 20.63 | 2.65 |
| Group 4 | 32.13 | 8.44 |

### EXAMPLE 4

The purpose of this study was to evaluate the renoprotective effects of hKDC or hMSC delivered locally under the renal capsule of an STZ SCID mouse model of diabetes mellitus.

### Study design

**Table 11: Experimental design.**

| Group Number | Number of animals | Description |
|---|---|---|
| 1 | 10 | Untreated |
| 2 | 14 | STZ + Vehicle |
| 3 | 14 | STZ + le6 hMSC, Lot# R012910, Passage 5 |
| 4 | 14 | STZ + 1e6 hKDCs, Lot# K100108, Passage 5 |

### Animal Model

A total of 52 animals (Jackson Laboratory male NOD (NOD.CB17-Prkdc^{scid}/J) SCID muscularis, approximately 24 grams each) were used in this study for the induction and treatment of diabetes. Diabetes (defined as blood glucose level of ≥ 300mg/dL) was induced by i.p injection of STZ (dissolved in 0.109 mol/L or 3.2 % (w/v) buffered sodium citrate at 10mg/ml concentration). Injections of *STZ* at *50* mg/Kg dose were given for five consecutive days (Group 2-3).

### Dose Preparation

On the day of cell treatment, Group 1 animals (Control Group) were surgically opened and closed with no cell treatment. Group 2 animals were surgically opened and their left kidneys were injected with saline. Groups 3 and 4 animals were surgically opened and their left kidneys were implanted with cells (1e⁶ hMSC for Group 3 and 1e⁶ hKDC for Group 4) (See Table 11 for treatment group assignments).

### Terminal Procedures

At day 7 post cell treatment, blood glucose levels were measured via tail vein using glucometer (OneTouch Ultra, Lifescan).

### Results

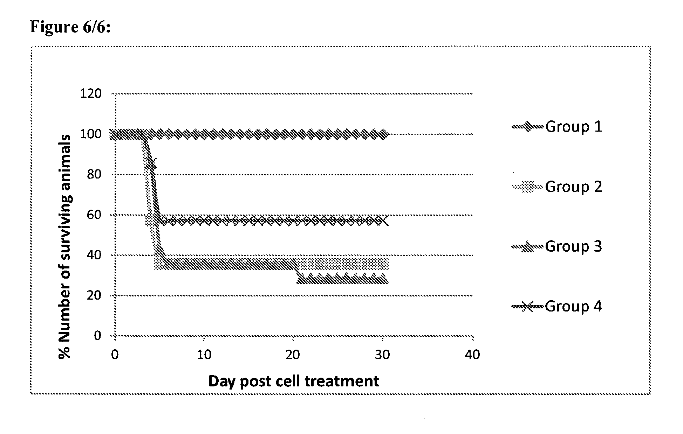

On day 7 post cell treatment, all animals were hypoglycemic with a blood glucose value of >300 mg/dl. Blood glucose values remained elevated throughout the duration of the study. On day 30 post cell treatment, 100% of Group 1 animals survived the duration of the study. In contrast, only 29% of the animals in Group 2, 36% of the animals in Group 3 and 57% of the animals in Group 4 survived the duration of the study (Figure 6).

### Conclusion

Previously, we showed that local administration of hKDCs protects the kidney from diabetic injury in an STZ model of diabetes. In this study, we showed that local, subcapsular administration of 1.0x10⁶ hKDCs promotes STZ induced diabetic animal survival.

## Claims

1. A population of human kidney derived cells for use in a method for treating diabetic nephropathy comprising administering a therapeutic amount of 0.18 x 10⁶ to 1.00 x 10⁶ of the human kidney derived cells to a mammalian subject; wherein the human kidney-derived cells are capable of self-renewal and expansion in culture; wherein the human kidney derived cells are positive for the cell surface marker expression of HLA I and CD44, and for the gene expression of Oct-4, Pax-2, and WT1; and wherein the human kidney derived cells are negative for the cell surface marker expression of CD133, and the gene expression of Wnt-4.

2. The population of human kidney derived cells for use in a method of claim 1, wherein the human kidney derived cells are additionally positive for the gene expression of BMP7, BMP2, GDF5, EpoR and Rex-1, and negative for the gene expression of Sox2, FGF4, and hTert.

3. The population of human kidney derived cells for use in a method of claim 1 or claim 2, wherein the human kidney derived cells are additionally positive for the cell-surface marker expression of CD24, CD29, CD49c, CD73, CD166, and SSEA-4, and negative for the cell-surface marker expression of HLA II, CD31, CD34, CD45, CD56, CD80, CD86, CD104, CD105, CD117, CD133, CD138, and CD141.

4. The population of human kidney derived cells for use in a method of any one of claims 1 to 3, wherein the human kidney derived cells are administered locally to a kidney of the mammal by a method selected from the group consisting of transplanting, implanting, injecting, and infusing.

5. The population of human kidney derived cells for use in a method of any one of claims 1 to 4 wherein the human kidney derived cells are administered under a kidney capsule of said mammal.

6. The population of human kidney derived cells for use in a method of claim 5, wherein the human kidney derived cells are administered under the kidney capsule by injection.

7. The population of human kidney derived cells for use in a method of claim 5, wherein the cells are administered locally to the kidney capsule by implanting.

8. The population of human kidney derived cells for use in a method of any one of claims 1 to 4, wherein the human kidney derived cells are administered to a kidney cortex of said mammal.

9. The population of human kidney derived cells for use in a method of claim 8, wherein the human kidney derived cells are administered to the kidney cortex by injection.

10. The population of human kidney derived cells for use in a method of claim 8, wherein the human kidney derived cells are administered to the kidney cortex by implantation.

11. The population of human kidney derived cells for use in a method of any one of claims 1 to 10, wherein the human kidney derived cells are implanted into the patient with a suitable scaffold.

12. The population of human kidney derived cells for use in a method of claim 11, wherein the scaffold is prepared from natural polymers, synthetic polymers or combinations thereof, and wherein the polymers are biodegradable and biocompatible.

13. The population of human kidney derived cells for use in a method of claim 11 or claim 12, wherein the scaffold is a foam.

14. The population of human kidney derived cells for use in a method of any one of claims 1 to 13, wherein the human kidney derived cells are administered in sequence with or co-administered with one or more biologically active agents, such as antithrombogenic agents, anti-apoptotic agents, anti-inflammatory agents, immunosuppressants, antioxidants, or eprodisate or triptolide.

## Patentansprüche

1. Population von aus menschlicher Niere stammenden Zellen zur Verwendung bei einem Verfahren zur Behandlung diabetischer Nephropathie, bei dem man eine therapeutische Menge von 0,18 x 10⁶ bis 1,00 x 10⁶ der aus menschlicher Niere stammenden Zellen einem Säugerindividuum verabreicht; wobei die aus menschlicher Niere stammenden Zellen zur Selbsterneuerung und Expansion in Kultur fähig sind; wobei die aus menschlicher Niere stammenden Zellen positiv für die Zelloberflächen-Marker-Expression von HLA I und CD44 und für die Genexpression von Oct-4, Pax-2 und WT1 sind; und wobei die aus menschlicher Niere stammenden Zellen negativ für die Zelloberflächen-Marker-Expression von CD133 und die Genexpression von Wnt-4 sind.

2. Population von aus menschlicher Niere stammenden Zellen zur Verwendung bei einem Verfahren nach Anspruch 1, wobei die aus menschlicher Niere stammenden Zellen zusätzlich positiv für die Genexpression von BMP7, BMP2, GDF5, EpoR und Rex-1 und negativ für die Genexpression von Sox2, FGF4 und hTert sind.

3. Population von aus menschlicher Niere stammenden Zellen zur Verwendung bei einem Verfahren nach Anspruch 1 oder Anspruch 2, wobei die aus menschlicher Niere stammenden Zellen zusätzlich positiv für die Zelloberflächen-Marker-Expression von CD24, CD29, CD49c, CD73, CD166 und SSEA-4 und negativ für die Zelloberflächen-Marker-Expression von HLA II, CD31, CD34, CD45, CD56, CD80, CD86, CD104, CD105, CD117, CD133, CD138 und CD141 sind.

4. Population von aus menschlicher Niere stammenden Zellen zur Verwendung bei einem Verfahren nach einem der Ansprüche 1 bis 3, wobei die aus menschlicher Niere stammenden Zellen einer Niere des Säugers lokal mit einem aus der aus Transplantieren, Implantieren, Injizieren und Infundieren bestehenden Gruppe ausgewählten Verfahren verabreicht werden.

5. Population von aus menschlicher Niere stammenden Zellen zur Verwendung bei einem Verfahren nach einem der Ansprüche 1 bis 4, wobei die aus menschlicher Niere stammenden Zellen unter eine Nierenkapsel des Säugers verabreicht werden.

6. Population von aus menschlicher Niere stammenden Zellen zur Verwendung bei einem Verfahren nach Anspruch 5, wobei die aus menschlicher Niere stammenden Zellen unter die Nierenkapsel durch Injektion verabreicht werden.

7. Population von aus menschlicher Niere stammenden Zellen zur Verwendung bei einem Verfahren nach Anspruch 5, wobei die Zellen der Nierenkapsel lokal durch Implantieren verabreicht werden.

8. Population von aus menschlicher Niere stammenden Zellen zur Verwendung bei einem Verfahren nach einem der Ansprüche 1 bis 4, wobei die aus menschlicher Niere stammenden Zellen einer Nierenrinde des Säugers verabreicht werden.

9. Population von aus menschlicher Niere stammenden Zellen zur Verwendung bei einem Verfahren nach Anspruch 8, wobei die aus menschlicher Niere stammenden Zellen der Nierenrinde durch Injektion verabreicht werden.

10. Population von aus menschlicher Niere stammenden Zellen zur Verwendung bei einem Verfahren nach Anspruch 8, wobei die aus menschlicher Niere stammenden Zellen der Nierenrinde durch Implantation verabreicht werden.

11. Population von aus menschlicher Niere stammenden Zellen zur Verwendung bei einem Verfahren nach einem der Ansprüche 1 bis 10, wobei die aus menschlicher Niere stammenden Zellen in den Patienten mit einem geeigneten Gerüst implantiert werden.

12. Population von aus menschlicher Niere stammenden Zellen zur Verwendung bei einem Verfahren nach Anspruch 11, wobei das Gerüst aus natürlichen Polymeren, synthetischen Polymeren oder Kombinationen davon hergestellt wird und wobei die Polymere biologisch abbaubar und biologisch verträglich sind.

13. Population von aus menschlicher Niere stammenden Zellen zur Verwendung bei einem Verfahren nach Anspruch 11 oder Anspruch 12, wobei es sich bei dem Gerüst um einen Schaum handelt.

14. Population von aus menschlicher Niere stammenden Zellen zur Verwendung bei einem Verfahren nach einem der Ansprüche 1 bis 13, wobei die aus menschlicher Niere stammenden Zellen mit einem oder mehreren biologisch wirksamen Mitteln, wie antithrombogenen Mitteln, anti-apoptotischen Mitteln, Entzündungshemmern, Immunsuppressiva, Antioxidantien oder Eprodisat oder Triptolid, der Reihe nach oder gleichzeitig verabreicht werden.

## Revendications

1. Population de cellules dérivées de rein humain pour utilisation dans un procédé pour traiter la néphropathie diabétique comprenant l'administration d'une quantité thérapeutique de 0,18 x 10⁶ à 1,00 x 10⁶ des cellules dérivées de rein humain à un sujet mammifère ; les cellules dérivées de rein humain étant capables d'autorenouvellement et d'expansion en culture ; les cellules dérivées de rein humain étant positives pour l'expression de marqueur de surface cellulaire de HLA I et CD44, et pour l'expression génique de Oct-4, Pax-2, et WT1 ; et les cellules dérivées de rein humain étant négatives pour l'expression de marqueur de surface cellulaire de CD133, et l'expression génique de Wnt-4.

2. Population de cellules dérivées de rein humain pour utilisation dans un procédé de la revendication 1, les cellules dérivées de rein humain étant en outre positives pour l'expression génique de BMP7, BMP2, GDF5, EpoR et Rex-1, et négatives pour l'expression génique de Sox2, FGF4, et hTert.

3. Population de cellules dérivées de rein humain pour utilisation dans un procédé de la revendication 1 ou la revendication 2, les cellules dérivées de rein humain étant en outre positives pour l'expression de marqueur de surface cellulaire de CD24, CD29, CD49c, CD73, CD 166, et SSEA-4, et négatives pour l'expression de marqueur de surface cellulaire de HLA II, CD31, CD34, CD45, CD56, CD80, CD86, CD104, CD105, CD117, CD133, CD138, et CD141.

4. Population de cellules dérivées de rein humain pour utilisation dans un procédé de l'une quelconque des revendications 1 à 3, les cellules dérivées de rein humain étant administrées localement à un rein du mammifère par un procédé choisi dans le groupe constitué d'une transplantation, implantation, injection et perfusion.

5. Population de cellules dérivées de rein humain pour utilisation dans un procédé de l'une quelconque des revendications 1 à 4, les cellules dérivées de rein humain étant administrées sous une capsule rénale dudit mammifère.

6. Population de cellules dérivées de rein humain pour utilisation dans un procédé de la revendication 5, les cellules dérivées de rein humain étant administrées sous la capsule rénale par injection.

7. Population de cellules dérivées de rein humain pour utilisation dans un procédé de la revendication 5, les cellules étant administrées localement à la capsule rénale par implantation.

8. Population de cellules dérivées de rein humain pour utilisation dans un procédé de l'une quelconque des revendications 1 à 4, les cellules dérivées de rein humain étant administrées à un cortex rénal dudit mammifère.

9. Population de cellules dérivées de rein humain pour utilisation dans un procédé de la revendication 8, les cellules dérivées de rein humain étant administrées au cortex rénal par injection.

10. Population de cellules dérivées de rein humain pour utilisation dans un procédé de la revendication 8, les cellules dérivées de rein humain étant administrées au cortex rénal par implantation.

11. Population de cellules dérivées de rein humain pour utilisation dans un procédé de l'une quelconque des revendications 1 à 10, les cellules dérivées de rein humain étant implantées dans le patient avec un échafaudage adapté.

12. Population de cellules dérivées de rein humain pour utilisation dans un procédé de la revendication 11, l'échafaudage étant préparé à partir de polymères naturels, de polymères synthétiques ou de combinaisons de ceux-ci, et les polymères étant biodégradables et biocompatibles.

13. Population de cellules dérivées de rein humain pour utilisation dans un procédé de la revendication 11 ou la revendication 12, l'échafaudage étant une mousse.

14. Population de cellules dérivées de rein humain pour utilisation dans un procédé de l'une quelconque des revendications 1 à 13, les cellules dérivées de rein humain étant administrées en séquence avec ou co-administrées avec un ou plusieurs agents biologiquement actifs, tels que des agents antithrombogéniques, des agents anti-apoptotiques, des agents anti-inflammatoires, des immunosuppresseurs, des antioxydants, ou l'éprodisate ou le triptolide.
